Europäisches Patentamt

European Patent Office

Office européen des brevets

(11) Veröffentlichungsnummer: **0 309 787 B1**

(12) **EUROPÄISCHE PATENTSCHRIFT**

(45) Veröffentlichungstag der Patentschrift: **17.06.92**

(51) Int. Cl.5: **A61K 37/02**, A61K 35/16, C07K 3/28, C12N 5/00

(21) Anmeldenummer: **88114592.4**

(22) Anmeldetag: **07.09.88**

(54) **Verfahren zur Herstellung eines hochreinen, virussicheren, biologisch aktiven Transferrinpräparates.**

(30) Priorität: **01.10.87 DE 3733181**

(43) Veröffentlichungstag der Anmeldung:
**05.04.89 Patentblatt 89/14**

(45) Bekanntmachung des Hinweises auf die Patenterteilung:
**17.06.92 Patentblatt 92/25**

(84) Benannte Vertragsstaaten:
**AT BE CH DE ES FR GB GR IT LI LU NL SE**

(56) Entgegenhaltungen:

**THE JOURNAL OF BIOLOGICAL CHEMISTRY, Band 242, Nr. 10, 25. Mai 1967, Seiten 2507-2513, US; W.E. ROOP et al.: "Purification and properties of human transferrin C and a slow moving genetic variant"**

**VOX SANG, Band 5, 1960, Seiten 403-415; P. KISTLER et al.: "Humanes Siderophilin: Isolierung mittels Rivanol aus Blutplasma und Plasmafraktionen, analytische Bestimmung und Kristallisation"**

(73) Patentinhaber: **BIOTEST PHARMA GMBH Landsteiner Strasse 5 W-6072 Dreieich(DE)**

(72) Erfinder: **Bethke, Ulf, Dr. Westend 10 W-6113 Babenhausen 3(DE)**
Erfinder: **Kothe, Norbert, Dr. Friedrich-Ebert-Strasse 21 W-6242 Kronberg(DE)**
Erfinder: **Rudnick, Dieter, Dr. Görlitzer Strasse 16 W-6074 Rödermark(DE)**
Erfinder: **Möller, Wolfgang, Dr. Graf von Stauffenbergstrasse 32 W-6370 Oberursel/Ts(DE)**
Erfinder: **Kloft, Michael, Dr. Kollwitzweg 27 W-6100 Darmstadt(DE)**

(74) Vertreter: **Beil, Hans Christoph, Dr. et al Beil, Wolff und Beil Rechtsanwälte Adelon-strasse 58 W-6230 Frankturt am Main 80(DE)**

EP 0 309 787 B1

CHEMICAL ABSTRACTS, Band 99, Nr. 26, 26. Dezember 1983, Seite 381, Zusammenfassung Nr. 218552x, Columbus, Ohio, US; A.M. PRINCE et al.: "Inactivation of hepatitis B and non-A, non-B viruses by combined use of Tween 80, beta-propiolactone, and ultraviolet irradiation", & THROMB. HAEMOSTASIS 1983, 50(2), 534-6

CHEMICAL ABSTRACTS, Band 97, Nr. 23, 6. Dezember 1982, Seite 299, Zusammenfassung Nr. 195285e, Columbus, Ohio, US; LABORATORY OF OUALITY CONTROL IN MEDICAL TECHNOLOGY: "Purification and identification of human plasma transferrin", & ZHONGHUA YIXUE JIANYAN ZAZHI 1981, 4(1), 25-9

## Beschreibung

Die Erfindung betrifft ein Verfahren zur Herstellung eines hochreinen, virussicheren, biologisch aktiven Transferrinpräparates aus transferrinhaltigen Plasmafraktionen.

Transferrin ist das Eisentransportprotein in menschlichem und tierischem Plasma und dort in einer Konzentration von ca. 2,5 g/l enthalten. Die Hauptfunktion von Transferrin ist seine Fähigkeit, spezifisch dreiwertiges Eisen zu binden. In dieser Form wird das Eisen nach der Resorption in Dünndarm bzw. nach der Übernahme vom Eisenspeicher Ferritin zu anderen Geweben transportiert.

Weitere Funktionen des Transferrins stellen seine Beteiligung an der Infektabwehr, sein positiver Einfluß auf das Zellwachstum sowie sein möglicher Einsatz als Diagnostikum dar (K. Theobald, W. König, DMW 110 (1985) 41, 1581) (1).

Auf Grund dieses vielfältigen Wirkunsspektrums erscheint es sinnvoll, ein hochreines, natives Transferrin in größeren Menge für therapeutische und diagnostische Zwecke herzustellen. Hierbei ist von essentieller Bedeutung, daß die empfindliche biologische Aktivität des Transferrins, meßbar als Eisenbindungskapazität, möglichst vollständig erhalten bleibt.

Wie für alle aus menschlichem Plasma hergestellten Proteinfraktionen, besteht auch hier die Gefahr einer Übertragung von Infektionen wie Hepatitis B, Hepatitis Non-A/Non-B und AIDS.

Deshalb muß ein Verfahren zur Herstellung von Transferrin einen hochwirksamen Schritt zur Abtrennung oder Inaktivierung infektiöser Viren enthalten.

Für die Sterilisation von Plasmaproteinen sind heute im wesentlichen drei Verfahren bekannt: Die Hitzeinaktivierung im feuchten und trockenen Zustand, die Sterilisation mit Detergentien und die kombinierte Behandlung mit $\beta$-Propiolacton und UV-Bestrahlung (EPA 0 142 059 (2); EPA 0 099 445 (3); DE 3 033 932 C 2 (4)).

Die Hitzeinaktivierung empfindlicher Plasmaproteine ist nur unter Zusatz von Stabilisatoren durchführbar, die unter Umständen auch Viren vor der Inaktivierung schützen. Deshalb kann diese Methode nicht in jedem Fall als sicher angesehen werden.

Im Gegensatz dazu ist die Behandlung mit Detergentien oder $\beta$-Propiolacton/UV-Bestrahlung ohne Zusatz von Stabilisatoren möglich und hat sich als effektiver Schritt zur Virusinaktivierung erwiesen. ((3); A. M. Prince et al.: Rev. Infect. Dis. 5 (1983) 1, 92 (5)).

Für die Herstellung von Transferrin sind in der Literatur einige Verfahren beschrieben. Ausgehend von Plasma oder Cohn-Paste IV werden verschiedene Fällungs- und Adsorptionsschritte angewandt. So beschreiben zum Beispiel Roop W. E. und Putnam F. W.: J. Biol. Chem. 242, 2507 (1967) (6), ein Verfahren, das ausgehend von Humanplasma auf einer Kombination von Rivanol-Fällung, Ammonsulfatfällung, Ausschluß- und Ionenaustauschchromatographie basiert.

Kistler et al. (Vox Sang. 5, 403 (1960) (7)) beschreiben ein ähnliches Verfahren, das Rivanol- und Alkoholfällungen kombiniert. Die Reinheit der so hergestellten Transferrin-Fraktion beträgt 85 %, die weitere Aufreinigung erfolgt durch Kristallisation.

Ausgehend von Paste IV (Cohn-Alkohol-Fällung) reinigen J. K. Inman et al. (Vox Sang. 6, 34 (1961) (8)) Transferrin über eine Kombination von Alkohol sowie Calciumhydroxid-, Zinkacetatfällung.

Die Endaufreinigung erfolgt durch batchweise Adsorption an Kationen- und Anionenaustauscher.

Die Reinheit des Produktes beträgt ca. 93 %.

Weitere in der Literatur beschriebene Herstellverfahren basieren auf Kombinationen von Fällungs- und Adsorptionsschritten.

Alle bekannten Verfahren weisen entscheidende Nachteile auf. Zum einen sind Reinheit und Ausbeute oft unbefriedigend, zum anderen sind die Verfahren oft so aufwendig, daß sie für eine wirtschaftliche, großtechnische Herstellung nicht geeignet sind.

Entscheidender Nachteil aller Verfahren ist das Fehlen virusinaktivierender Schritte, wodurch eine therapeutische Anwendung mit dem Risiko einer Infektionsübertragung behaftet ist.

Lediglich J. K. Inman (8) beschreibt ein Hitzeinaktivierungsverfahren, das jedoch auf eisengesättigtes Transferrin beschränkt ist. Da für die therapeutische Anwendung auch eisenfreies Transferrin erforderlich ist, kann diese Methode nicht allgemein verwendet werden.

Wie eigene Messungen zeigen (Abb. 1), entstehen auch in eisengesättigten Transferrinlösungen durch die Hitzebehandlung Aggregate, die bei der intravenösen Applikation zu Unverträglichkeitsreaktionen führen können.

**Tabelle 1:**

| | MG > 80 000 D vor Hitzebehandlung | MG > 80 000 D nach Hitzebehandlung 10 h 60°C |
|---|---|---|
| eisengesättigtes Transferrin | 10 % | 61 % |
| eisenfreies Transferrin | 4 % | total denaturiert |

Die bei Imman et al (8) beschriebene Viskositätserhöhung bestätigt diese Befunde.

Der Erfindung liegt die Aufgabe zugrunde, ein einfaches, im großtechnischen Maßstab durchführbares Verfahren zur Herstellung von biologisch aktivem Transferrin bereitzustellen, bei dem ein Transferrin erhalten wird, das sich durch hohe Reinheit, Nativität und Abwesenheit von infektiösen Viren auszeichnet und für in-vitro- und in-vivo-Anwendungen geeignet ist.

Je nach Verwendungszweck kann das Transferrin eisenfrei oder eisehaltig hergestellt werden.

Diese Aufgabe wird erfindungsgemäß bei einem gattungsgemäßen Verfahren durch die kennzeichnenden Merkmale des Anspruchs 1 gelöst.

Zwar hat man, wie vorstehend ausgeführt, mit Hilfe von Sterilisationsverfahren, wie Behandlung mit Detergentien oder $\beta$-Propiolacton/UV-Bestrahlung, bei der Virusinaktivierung von Blutplasma recht gute Erfolgt erzielt, jedoch war nicht vorauszusehen, daß bei einem Transferrinpräparat mit seiner hochempfindlichen biologischen Aktivität beim Sterilisieren diese biologische Aktivität erhalten bleiben würde. Es war vielmehr zu erwarten, daß das Transferrin denaturiert werden würde.

Um ein hochreines Transferrinpräparat zu erhalten, ist es außerdem erforderlich, die Begleitproteine abzutrennen. Man fand, daß mit Hilfe der Ionenaustausch-Chromatographie eine unerwartet wirksame Abtrennung erfolgt. Man fand außerdem, daß es wichtig ist, die Reinigung über Ionenaustauscher nach der Sterilisation vorzunehmen, weil dann auch eventuell bei der Sterilisation denaturierte oder modifizierte Proteine am Ionenaustauscher abgetrennt werden.

Als Ausgangsmaterial verwendet man eine transferrinhaltige Fraktion aus Plasma, gewonnen durch Fällmethoden, z.B. Alkoholfraktionierung nach Cohn, oder chromatographische Fraktionierung, wie z.B. über Ionenaustauscher.

Diese Fraktion wird durch Behandlung mit einem Fällungsmittel für Immunglobuline, wie es z.B. aus (6) oder (7) bekannt ist, wie z.B. Ammonsulfat, Polyethylenglycol (PEG) oder 2-Ethoxy-6,9-diaminoacridin-DL-Lactat (Rivanol) von Immunglobulinen weitestgehend befreit.

Nach Abtrennen des Niederschlages wird der Überstand mittels Gelfiltration oder Diafiltration an Ultrafiltrationsmembranen, die als Flach- oder Hohlfasermembranen eingesetzt werden, von dem Fällungsmittel befreit, auf eine niedrige Ionenkonzentration von 0,005-0,5 Mol pro Liter und auf eine Proteinkonzentration von 10-60 g/l eingestellt.

Diese Lösung wird bei einem pH-Wert von 6 - 9 mit $\beta$-Propiolacton in einer Konzentration von 0.01 - 0.5 % behandelt.

Nach Verdünnen auf einen Proteinwert von 2 - 25 g/l wird diese Lösung mittels eines Durchfluß-UV-Gerätes bei 254 nm bestrahlt.

Anstelle der $\beta$PL/UV-Behandlung kann diese Lösung mit Detergentien, z.B. Tri-n-butylphosphat in einer Menge von 0,05 bis 5% und Natriumcholat in einer Menge von 0,03 bis 0,4% bzw. Sorbimacrogololeat (Tween 80®) in einer Menge von 0,1 bis 5%, sterilisiert werden.

Die weitere Aufreinigung des Transferrins, insbesondere die Abtrennung von Albumin und anderer Begleitproteine, erfolgt durch Ionenaustausch-Chromatographie.

Die Ionenstärke ist so gewählt, daß alle Proteine, außer Transferrin, adsorbiert werden.

Dieser Verfahrensschritt kann säulenchromatographisch oder batchweise durchgeführt werden.

Als Adsorptionsmedien eignen sich handelsübliche Anionenaustauscher mit DEAE-, QAE-/QMA-Gruppen, z. B. DEAE-Trisacryl-LS®, QMA-Accell®, Q-Sepharose®, DEAE-Sephadex®, AEA-Euperegit® usw.

Die auf diese Weise gewonnene verdünnte Transferrin-Fraktion wird zur Entfernung von Puffersubstanzen gegen physiologisch verträgliche Salzlösungen diafiltriert bzw. gelfiltriert und mittels Ultrafiltration ankonzentriert.

Gegebenenfalls kann bei diesem Schritt das Eisen entfernt werden, indem man einen pH-Wert von 4.0 - 7.0 einstellt, mit einem Komplexbildner, wie z. B. Ethylendiamintetraessigsäure, Natriumsalz (EDTA), versetzt und unter diesen Bedingungen die Diafiltration bzw. Gelfiltration durchführt.

Ein nach dem erfindungsgemäßen Verfahren hergestelltes Transferrinpräparat zeichnet sich durch nachfolgend aufgeführte Eigenschaften aus:

Tabelle 2

| Protein: | 150 g/l |
|---|---|
| Transferrin | 150 g/l |
| IgG | 0.025 g/l |
| IgA | 0 g/l |
| IgM | 0 g/l |
| Albumin | 0 g/l |
| Celluloseacetat-Folienelektrophorese (CAF) | 100 % $\beta$-Globulin |
| proteolytische Aktivität | 17 U/l |
| Plasmin | negativ |
| Plasminogen | negativ |
| Thrombin | negativ |
| PKA | negativ |
| Komplement-Bindungsreaktion | 2 $\mu$l Komplement/mg Protein |
| Eisenbindungskapazität | 80% |

Die Effektivität des Sterilisationsverfahrens mit $\beta$-Propiolacton und UV-Bestrahlung wurde mit Hilfe von Bakteriophagen ermittelt. Die Reduktion um 7.5 $\log_{10}$ für die Bakterienviren Phi-X 174, Phi-e und Kappa entspricht einer ausreichenden Virussicherheit für Hepatitis B, Non-A/Non-B und AIDS.

Wie bereits vorstehend ausgeführt, zeigte sich überraschenderweise, daß das erfindungsgemäße Verfahren zur Herstellung eines hochreinen, sterilisierten, virussicheren Transferrin die biologische Aktivität des Proteins nicht beeinflußt.

Wird beispielsweise die transferrinhaltige Paste IV nach Cohn, in der die Aktivität noch ca. 80 % beträgt, als Ausgangsmaterial eingesetzt, so findet man im gereinigten Transferrin diese Aktivität wieder.

Tabelle 3

| | Fe-Bindungskapazität |
|---|---|
| Ausgang | 83 % |
| nach Sterilisation | 80 % |
| Endprodukt | 80 % |

Ein nach dem erfindungsgemäßen Verfahren hergestelltes Transferrinpräparat wurde als Wachstumsfaktor in Nährkulturmedium eingesetzt.

Im allgemeinen werden transformierte und nicht transformierte Säugetierzellen in einem Medium gezüchtet, das heterologes Serum, z. B. Kälberserum, enthält. Dieses Serum enthält natürlicherweise den Wachstumsfaktor Transferrin. In vielen Fällen ist es jedoch wünschenswert, spezielle bei der Produktion von Therapeutika zur Anwendung am Menschen, ein definiertes Wachstumsmedium einzusetzen, das frei von heterologen Proteinen ist.

Unabdingbar für die Vermehrung von Säugerzellen ist der Wachstumsfaktor Transferrin (Ham, R. G., et al.: Methods in Enzymology, Vol. LVIII, 44 (1979), Academic Press (9)).

Auch in serumfreien Medien muß deshalb Transferrin zugesetzt werden, wobei sich für obengenannte Indikationen ein Transferrin humanen Ursprungs anbietet, um Sensibilisierungen zu vermeiden.

Für diesen Einsatz ist die Verwendung eines sterilisierten, humanen, biologisch aktiven Transferrins notwendig, um eine virale Kontamination auszuschließen. Dies gilt insbesondere dann, wenn ein Produkt für den therapeutischen Einsatz am Menschen hergestellt werden soll.

Zu einem serumfreien Medium (DME/F 12) wurde ein nach dem erfindungsgemäßen Verfahren hergestelltes Transferrin zugesetzt und an zwei Hybridomlinien auf seine Wirksamkeit geprüft.

Überraschenderweise zeigte sich, daß auch das nach dem erfindungsgemäßen Verfahren gereinigte und sterilisierte Transferrin als Wachstumsfaktor geeignet ist (siehe Abb. 2).

## Tabelle 4

### Antikörperproduktion von Hybridom-Zellen in serumhaltigen und serumfreien Nährmedien

| Zeit d | Zusatz von 10% FCS | Zusatz von 10 mg/l Transferrin | ohne Zusatz |
|---|---|---|---|
| 1 | 4 µg/ml | 4 µg/ml | 4 µg/ml |
| 2 | 11 " | 12 " | 11 " |
| 3 | 14 " | 17 " | |
| 4 | 17 " | 20 " | Zelltod |
| 5 | 21 " | 27 " | |

Wie aus Abbildung 3 und Tabelle 4 zu ersehen, liegen sowohl die Wachstumsraten als auch die Produktion von monoklonalen Antikörpern deutlich höher als in transferrinfreiem Medium und sind sogar gegenüber Kälberserum erhöht.

Im Tierexperiment an Mäusen und Meerschweinchen erwies sich die hochreine, virussichere Transferrinlösung bei intravenöser Anwendung als gut verträglich.

Die Erfindung wird durch folgende Beispiele erläutert:

Beispiel 1

1 kg Cohn-Paste IV wird im zehnfachen Volumen Puffer (Trishydroxyaminomethan, 0.02 Molar, pH 7.5) suspendiert und bei Raumtemperatur eine Stunden gerührt. Mit 1 N Natronlauge stellt man den pH-Wert auf 8.0 und gibt festes Ammonsulfat bis zu einer Konzentration von 2.5 Molar zu. Man rührt zwei Stunden und läßt über Nacht bei +4°C stehen.

Der Niederschlag wird durch Zentrifugation abgetrennt, der Überstand durch Filtration geklärt.

Mit Hilfe einer Ultrafiltrationseinheit (Membran Cut-off 10.000 Daltons) entfernt man das Fällungsmittel, stellt auf 0.020 Molar Trishydroxyaminomethan Ph 7.5 ein und konzentriert auf einen Proteingehalt von 40 g/l.

Diese Lösung wird mit 1 % Aktivkohle versetzt, klärfiltriert, der pH-Wert mit 1 N Salzsäure auf 7.5 eingestellt und mit 0.05 % β-Propiolacton versetzt.

Man hält den pH-Wert über vier Stunden konstant und läßt über Nacht bei +4°C stehen.

Nach Verdünnen mit 0.02 M Trispuffer, pH 7.5, auf einen Proteingehalt von 10 g/l führt man eine UV-Bestrahlung bei 254 nm in einer Durchflußapparatur durch. Die Lösung wird mit 1.6 g Kochsalz pro Liter versetzt und auf einen Anionenaustauscher DEAE-Trisacryl-LS aufgebracht.

Die durchlaufende Fraktion wird aufgefangen, auf die gewünschte Konzentration ankonzentriert und sterilfiltriert.

Zur Entfernung des gebundenen Eisens stellt man den pH-Wert dieser Transferrinlösung auf 6.0 und fügt EDTA im Überschuß zu.

EDTA und komplexiertes Eisen werden mit Hilfe einer Ausschlußchromatographiesäule, gefüllt mit Sephadex G-25, und äquilibriert mit 0,45% Kochsalz, entfernt

Die Proteinfraktion wird aufgefangen, auf die gewünschte Konzentration ankonzentriert und sterilfiltriert.

| Ausbeute: | 75 % |
|---|---|
| Celluloseacetat-Folienelektrophorese (CAF) | 100 % $\beta$-Globulin |
| Protein | 150 g/l |
| Transferrin | 150 g/l |
| Komplement-Bindungsreaktion | 2 $\mu$l Komplement/mg Protein |
| proteolytische Aktivität | 17 U/l |
| Eisenbindungskapazität | 80 % |

Beispiel 2

Man verfährt wie in Beispiel 1. Nach der UV-Bestrahlung der 1 %igen Lösung gibt man 2.9 g Kochsalz pro Liter Lösung dazu, stellt den pH-Wert auf 6.2 ein und versetzt mit 84 ml DEAE-Sephadex-A-50, das zuvor mit 0.05 M NaCl, 0.02 M Tris pH 6.2 äquilibriert wurde. Nach einer Adsorptionszeit von 2 h wird das Gel abgetrennt und mit einem Gelvolumen gewaschen. Die vereinigten Filtrate werden wie in Beispiel 1 beschrieben weiter aufgearbeitet.

| Ausbeute: | 78 % |
|---|---|
| CAF: | 95,5 % $\beta$-Globulin |
| Protein: | 78,3 g/l |
| Transferrin: | 75.5 g/l |
| Komplement-Bindungsreaktion: | 5 $\mu$l Komplement/mg Protein |
| proteolytische Aktivität: | 10 U/l |
| Eisenbindungskapazität: | 72 % |

Beispiel 3

Bei der Isolierung von IgG aus Serum mit Ionenaustausch-Chromatographie erhält man eine $\beta$-Globulin-reiche Fraktion, die als Ausgangsmaterial für die Transferrin-Herstellung eingesetzt werden kann. Die Fraktion ist wie folgt zusammengesetzt:
66.1 % $\beta$-Globulin, 6.0 % $\alpha$-Globulin, 14.5 g $\gamma$-Globulin, 13.4 % Albumin (Celluloseacetat-Folienelektrophorese (CAF)). Aus dieser Lösung läßt sich das Transferrin wie in Beispiel 1 beschrieben isolieren.

| Ausbeute: | 70 % |
|---|---|
| CAF: | 100 % $\beta$-Globulin |
| Protein: | 36.0 g/l |
| Transferrin: | 35.2 g/l |
| Komplement-Bindungsreaktion: | 8 $\mu$l Komplement/mg Protein |
| Proteolytische Aktivität: | 29 U/l |
| Eisenbindungskapazität: | 89 % |

Beispiel 4

Gemäß Beispiel 1 läßt sich auch aus einem Gemisch der verschiedenen Ausgangsmaterialien, wie sie in den Beispielen 1 und 3 verwendet werden, ein Transferrin gewinnen.
Zu einer Suspension von 1 kg Paste IV in 10 l 0.02 M Trispuffer pH 7.5 fügt man 3.5 l der in Beispiel 3 gewonnenen $\beta$-Globulin-Fraktion zu. Die Aufarbeitung erfolgt wie in Beispiel 1 beschrieben.

| Ausbeute: | ca. 75 % |
|---|---|
| CAF: | 100 % $\beta$-Globulin |
| Protein: | 106 g/l |
| Transferrin: | 101 g/l |
| Komplement-Bindungsreaktion | 8 $\mu$l Komplement/mg Protein |
| Proteolytische Aktivität: | 25 U/l |
| Eisenbindungskapazität: | 85 % |

**Patentansprüche**

**Patentansprüche für folgende Vertragsstaaten : AT, BE, CH, DE, FR, GB, IT, LI, LU, NL, SE**

1.  Verfahren zur Herstellung eines hochreinen, virussicheren, biologisch aktiven Transferrinpräparates aus transferrinhaltigen Plasmafraktionen, dadurch gekennzeichnet, daß man aus der transferrinhaltigen Fraktion

    a) die $\gamma$-Globuline mittels einer Fällungsreaktion entfernt;

    b) aus der rückständigen Flüssigkeit die Fällungsmittel durch Ultra- oder Gelfiltration entfernt;

    c) die Flüssigkeit auf eine Ionenkonzentration von 0,005 - 0,5 Molar und

    d) eine Protein-Konzentration von 10 - 60 g/l einstellt;

    e) mit 0,01-0,5 % $\beta$-Propiolacton bei pH 6,0-9,0 behandelt, die Proteinkonzentration auf 2-25 g/l einstellt und die Lösung einer UV-Bestrahlung bei 254 nm unterzieht oder das Produkt der Stufe d) mit 0,05 bis 5% Tri-n-butylphosphat und 0,03 bis 0,4% Natriumcholat bzw. 0,1 bis 5% Sorbimacrogololeat behandelt;

    f) die Lösung einer Ionenaustauschchromatographie unterwirft, wobei die Ionenstärke so gewählt wird, daß alle Proteine außer Transferrin adsorbiert werden und

    g) das Transferrin in an sich bekannter Weise ankonzentriert und sterilfiltriert.

2.  Verfahren nach Anspruch 1, dadurch gekennzeichnet, daß man im Anschluß an Stufe f) gebundenes Eisen mit Komplexbildner bindet und mit Hilfe einer Ultra- oder Gelfiltration entfernt.

3.  Hochreines, virussicheres, biologisch aktiven Transferrinpräparat, hergestellt nach Anspruch 1 oder 2.

4.  Verwendung eines Transferrinpräparates gemäß Anspruch 3 zur Herstellung eines Mittels für die intravenöse Anwendung sowie als Wachstumsfaktor in Nährkulturmedien von Säugetierzellen.

**Patentansprüche für folgende Vertragsstaaten : ES, GR**

1.  Verfahren zur Herstellung eines hochreinen, virussicheren, biologisch aktiven Transferrinpräparates aus transferrinhaltigen Plasmafraktionen, dadurch gekennzeichnet, daß man aus der transferrinhaltigen Fraktion

    a) die $\gamma$-Globuline mittels einer Fällungsreaktion entfernt;

    b) aus der rückständigen Flüssigkeit die Fällungsmittel durch Ultra- oder Gelfiltration entfernt;

    c) die Flüssigkeit auf eine Ionenkonzentration von 0,005 - 0,5 Molar und

    d) eine Protein-Konzentration von 10 - 60 g/l einstellt;

    e) mit 0,01-0,5 % $\beta$-Propiolacton bei pH 6,0-9,0 behandelt, die Proteinkonzentration auf 2-25 g/l einstellt und die Lösung einer UV-Bestrahlung bei 254 nm unterzieht oder das Produkt der Stufe d) mit 0,05 bis 5% Tri-n-butylphosphat und 0,03 bis 0,4% Natriumcholat bzw. 0,1 bis 5% Sorbimacrogololeat behandelt;

    f) die Lösung einer Ionenaustauschchromatographie unterwirft, wobei die Ionenstärke so gewählt wird, daß alle Proteine außer Transferrin adsorbiert werden und

    g) das Transferrin in an sich bekannter Weise ankonzentriert und sterilfiltriert.

2.  Verfahren nach Anspruch 1, dadurch gekennzeichnet, daß man im Anschluß an Stufe f) gebundenes Eisen mit Komplexbildner bindet und mit Hilfe einer Ultra- oder Gelfiltration entfernt.

3.  Verwendung eines Transferrinpräparates hergestellt nach dem Verfahren gemaß Anspruch 1 oder 2 zur Herstellung eines Mittels für die intravenöse Anwendung sowie als Wachstumsfaktor in Nährkulturme-

EP 0 309 787 B1

dien von Säugetierzellen.

**Claims**
**Claims for the following Contracting States : AT, BE, CH, DE, FR, GB, IT, LI, LU, NL, SE**

1.  A process for the preparation of a highly pure, virus resistant, biologically active transferrin preparation from plasma fractions containing transferrin, characterised in that from the transferrin-containing fraction
    a) the γ-globulins are removed by means of a precipitation reaction;
    b) the precipitating agent is removed from the residual liquid by ultrafiltration or gel filtration;
    c) the liquid is adjusted to an ion concentration of from 0.005 to 0.5 molar and
    d) to a protein concentration of from 10 to 60 g/l;
    e) and treated with from 0.01-0.5% of $\beta$-propiolactone at pH 6.0-9.0, the protein concentration is adjusted to 2-25 g/l and the solution is subjected to UV irradiation at 254 nm or the product from stage d) is treated with from 0.05 to 5% of tri-n-butylphosphate and from 0.03 to 0.4% of sodium cholate or from 0.1 to 5% of sorbimacrogol oleate;
    f) the solution is subjected to ion exchange chromatography, the ionic strength being chosen so that all the proteins are adsorbed with the exclusion of transferrin and
    g) the transferrin is concentrated and filtered sterile in known manner.

2.  A process according to Claim 1, characterised in that following Stage f), bound iron is bound with complex former and removed by means of an ultrafiltration or gel filtration.

3.  Highly pure, virus resistant, biologically active transferrin preparation prepared according to Claim 1 or Claim 2.

4.  Use of a transferring preparation according to Claim 3 for the preparation of an agent for intravenous use and as growth factor in nutrient culture media of mammalian cells.

**Claims for the following Contracting States : ES, GR**

1.  A process for the preparation of a highly pure, virus resistant, biologically active transferrin preparation from plasma fractions containing transferrin, characterised in that from the transferrin-containing fraction
    a) the γ-globulins are removed by means of a precipitation reaction;
    b) the precipitating agents are removed from the residual liquid by ultrafiltration or gel filtration;
    c) the liquid is adjusted to an ion concentration of from 0.005 - 0.5 molar and
    d) to a protein concentration of from 10 - 60 g/l
    e) and is treated with from 0.01-0.5% of $\beta$-propiolactone at pH 6.0-9.0, the protein concentration is adjusted to 2-25 g/l and the solution is subjected to UV irradiation at 254 nm or the product from Stage d) is treated with from 0.05 to 5% of tri-n-butylphosphate and from 0.03 to 0.4% of sodium cholate or from 0.1 to 5% of sorbimacrogol oleate;
    f) the solution is subjected to an ion exchange chromatography, the ionic strength being chosen so that all proteins with the exclusion of transferrin are adsorbed and
    g) the transferrin is concentrated and filtered sterile in known manner.

2.  A process according to Claim 1, characterised in that following Stage f), bound iron is bound with complex former and removed by means of ultrafiltration or gel filtration.

3.  Use of a transferrin preparation prepared according to the process of Claim 1 or Claim 2 for the preparation of an agent for intravenous use and as growth factor in nutrient culture media of mammalian cells.

**Revendications**
**Revendications pour les Etats contractants suivants : AT, BE, CH, DE, FR, GB, IT, LI, LU, NL, SE**

1.  Procédé de production d'une préparation de transferrine de haute pureté, exempte de virus, biologiquement active, à partir de fractions plasmatiques contenant de la transferrine, caractérisé en ce que:
    a) on élimine de la fraction contenant de la transferrine les γ-globulines à l'aide d'une réaction de précipitation ;

9

b) on élimine du liquide résiduel l'agent de précipitation, par ultrafiltration ou filtration sur gel ;

c) on ajuste le liquide à une concentration ionique de 0,005-0,5 M ; et

d) on ajuste la concentration des protéines à 10-60 g/litre ;

e) on traite avec 0,01-0,5 % de $\beta$-propiolactone à pH 6,0-9,0, on ajuste la concentration des protéines à 2-25 g/litre et on soumet la solution à un rayonnement UV à 254 nm, ou encore on traite le produit de l'étape d) avec 0,05 à 5 % de phosphate de tri-n-butyle et 0,03 à 0,4 % de cholate de sodium, ou avec 0,1 à 5 % d'oléate de sorbimacrogol ;

f) on soumet la solution à une chromatographie par échange d'ions en choisissant la force ionique de façon que toutes les protéines soient adsorbées, à l'exception de la transferrine ; et

g) on concentre et on filtre d'une manière stérile la transferrine, d'une manière connue en soi.

**2.** Procédé selon la revendication 1, caractérisé en ce qu'on fixe avec un complexant le fer fixé dans l'étape f) et on l'élimine par ultrafiltration ou filtration sur gel.

**3.** Préparation de transferrine de haute pureté, exempte de virus, biologiquement active, préparée selon la revendication 1 ou 2.

**4.** Utilisation d'une préparation de transferrine selon la revendication 3, pour la préparation d'un produit destiné à une administration intraveineuse et comme facteur de croissance dans des milieux de culture nutritifs de cellules de mammifères.

**Revendications pour les Etats contractants suivants : ES, GR**

**1.** Procédé de production d'une préparation de transferrine de haute pureté, exempte de virus, biologiquement active, à partir de fractions plasmatiques contenant de la transferrine, caractérisé en ce que:

a) on élimine de la fraction contenant de la transferrine les $\gamma$-globulines à l'aide d'une réaction de précipitation ;

b) on élimine du liquide résiduel l'agent de précipitation, par ultrafiltration ou filtration sur gel ;

c) on ajuste le liquide à une concentration ionique de 0,005-0,5 M ; et

d) on ajuste la concentration des protéines à 10-60 g/litre ;

e) on traite avec 0,01-0,5 % de $\beta$-propiolactone à pH 6,0-9,0, on ajuste la concentration des protéines à 2-25 g/litre et on soumet la solution à un rayonnement UV à 254 nm, ou encore on traite le produit de l'étape d) avec 0,05 à 5 % de phosphate de tri-n-butyle et 0,03 à 0,4 % de cholate de sodium, ou avec 0,1 à 5 % d'oléate de sorbimacrogol ;

f) on soumet la solution à une chromatographie par échange d'ions en choisissant la force ionique de façon que toutes les protéines soient adsorbées, à l'exception de la transferrine ; et

g) on concentre et on filtre d'une manière stérile la transferrine, d'une manière connue en soi.

**2.** Procédé selon la revendication 1, caractérisé en ce qu'on fixe avec un complexant le fer fixé dans l'étape f) et on l'élimine par ultrafiltration ou filtration sur gel.

**3.** Utilisation d'une préparation de transferrine selon la revendication 3, pour la préparation d'un produit destiné à une administration intraveineuse et comme facteur de croissance dans des milieux de culture nutritifs de cellules de mammifères.